# EUROPÄISCHE PATENTSCHRIFT

(11) **EP 1 858 496 B1**
(45) Veröffentlichungstag und Bekanntmachung des Hinweises auf die Patenterteilung: **23.07.2008**
(21) Anmeldenummer: 06724947.4
(22) Anmeldetag: 06.03.2006
(51) Int. Cl.: A61K 9/70, A61K 31/485

(54) **TRANSDERMALES THERAPEUTISCHES SYSTEM ZUR VERABREICHUNG VON ANALGETIKA**
TRANSDERMAL THERAPEUTIC SYSTEM FOR THE ADMINISTRATION OF ANALGESICS
SYSTEME THERAPEUTIQUE TRANSDERMIQUE DESTINE A L'ADMINISTRATION D'ANALGESIQUES

(30) Priorität: 10.03.2005 DE 102005011517
(43) Veröffentlichungstag der Anmeldung: 28.11.2007
(73) Patentinhaber: GRUENENTHAL GMBH, 52078 Aachen (DE)
(72) Erfinder: KUGELMANN, Heinrich, 52068 Aachen (DE); BARTHOLOMÄUS, Johannes, 52080 Aachen (DE)
(74) Vertreter: Bülle, Jan
(86) Internationale Anmeldenummer: PCT/EP2006/060484
(87) Internationale Veröffentlichungsnummer: WO 2006/094961

(56) Entgegenhaltungen:
- EP-A- 0 484 543
- EP-A- 0 601 463
- EP-A- 1 174 137
- EP-A- 1 323 421

## Beschreibung

Die Erfindung betrifft ein transdermales therapeutisches System zur Verabreichung von Analgetika, bevorzugt von Buprenorphin, eines seiner pharmazeutisch verträglichen Salze oder Prodrugs, und ein Verfahren zu Herstellung eines solchen Systems.

Wirkstoffe können mit speziellen transdermalen therapeutischen Systemen durch die Haut systemisch verabreicht werden. Bei den transdermalen therapeutischen Systemen (TTS) kann hinsichtlich der Wirkstofffreisetzung zwischen membrangesteuerten Systemen (Reservoir-TTS) und matrixgesteuerten Systemen (Matrix-TTS) unterschieden werden.

Ein Reservoir-TTS ist üblicherweise ein auf der Haut selbstklebender, flacher Beutel, welcher die Wirkstoffe gelöst enthält. Der Hautseite zugewandt ist der Beutel mit einer halbdurchlässigen Membran ausgerüstet, welche die Wirkstofffreigabe steuert. Der Transport des Wirkstoffs in den Blutkreislauf beruht auf dem Gefälle des chemischen Potentials zwischen Wirkstoff-Reservoir und Blutkreislauf (Osmose). Ein Matrix-TTS umfasst üblicherweise einen wirkstoffhaltigen Klebstofffilm in einer ovalen, runden oder eckigen Form. Der Wirkstoff liegt üblicherweise gelöst oder suspendiert in der Klebeschicht vor, aus der die Wirkstofffreisetzung erfolgt. Hinsichtlich weiterer Einzelheiten kann beispielsweise auf T.K. Gosh, Transdermal and Topical Drug Delivery Systems Es Into Practice, CRC Press, 1997; R.O. Potts et al., Mechanisms of Transdermal Drug Delivery (Drugs and the Pharmaceutical Sciences), Marcel Dekker, 1997; und R. Gurny et al, Dermal and Transdermal Drug Delivery. New Insights and Perspectives, Wissenschaftliche VG., Stuttgart, 1998 verwiesen werden.

Damit ein Wirkstoff transdermal wirken kann, muss er in ausreichender Menge durch die Epidermis der Haut diffundieren und vom Blutkreislauf aufgenommen werden. Ein wichtiger begrenzender Faktor ist dabei die unterschiedliche Durchlässigkeit der Haut für verschiedene Wirkstoffe. Die transkutane Permeation des Wirkstoffs aus dem transdermalen therapeutischen System kann mitunter je nach chemischer Struktur und Lipophile des Wirkstoffs durch Zusatz geeigneter Hilfsstoffe verbessert werden. Durch die Zusammensetzung und Strukturierung der Wirkstoffmatrix und/oder Diffusionsmembran kann die Freisetzung des Wirkstoffs reguliert werden.

Neben den gewünschten pharmakokinetischen Parametern muss ein transdermales therapeutisches System eine ausreichende Hautverträglichkeit aufweisen. Einerseits dürfen die Inhaltsstoffe weder Allergien noch eine Kontaktdermatitis hervorrufen, andererseits sollte insbesondere auch das Aufbringen und Entfernen des Systems schonend erfolgen und keine Hautirritationen hervorrufen. Die Haftung des Systems auf der Haut muss demnach ausreichen, um eine dauerhafte Medikation zu gewährleisten, darf andererseits aber nicht zu stark sein, damit ein Entfernen nach der Anwendung ohne Schwierigkeiten möglich ist. Ferner sollte ein transdermales therapeutisches System, welches häufig für mehrere Tage vom Patienten angewendet wird, eine ausreichende Wasserbeständigkeit und eine ausreichende mechanische Beständigkeit aufweisen, damit es infolge äußerer Einflüsse nicht beschädigt oder unbeabsichtigt entfernt wird.

Ein Nachteil herkömmlicher transdermaler therapeutischer Systeme besteht darin, dass sie häufig nicht die gesamte Menge des enthaltenen Wirkstoffs innerhalb des für die Anwendung vom Hersteller empfohlenen Zeitintervalls freisetzen. So werden zum Beispiel aus einem kommerziell erhältlichen System mit einer Fläche von 2,5 cm², welches als Wirkstoff Scopolamin zur Prophylaxe der Reisekrankheit enthält und hinter das Ohr auf die Haut aufgebracht wird, drei Tage lang konstant etwa 2,5 µg/h Wirkstoff freigegeben. Dies sind insgesamt nur etwa 30% des im Depot insgesamt enthaltenen Scopolamins. Auf dem Markt befindliche Pflaster, welche Nitroglycerin enthalten und auf die Herzgegend der Brust appliziert werden, sind meist für eine Wirkstoff-Anwendung für 24 h konzipiert. Innerhalb dieses Zeitintervalls werden 5 bzw. 10 mg Wirkstoff konstant abgegeben. Das sind etwa 20% der im Reservoir befindlichen Menge an Nitroglycerin. Hinsichtlich weiterer Einzelheiten kann beispielsweise verwiesen werden auf K.H. Bauer et al., Lehrbuch der Pharmazeutischen Technologie, Wissenschaftliche VG, Stuttgart, 1999.

Die nach bestimmungsgemäßer Anwendung in diesen transdermalen therapeutischen Systemen verbleibende Restmenge des Wirkstoffs ist nicht unproblematisch. Einerseits verursacht sie zusätzliche Kosten, andererseits ist sie häufig auch aus ökologischer Sicht im Zusammenhang mit der sachgerechten Entsorgung derartiger Systeme von Nachteil.

Zusätzliche Probleme ergeben sich, wenn der im transdermalen therapeutischen System enthaltene Wirkstoff ein Missbrauchspotential aufweist, d.h. bei nicht bestimmungsgemäßer Anwendung zur Erzielung rauschartiger Zustände verwendet werden kann. Beispielsweise ist das Opioid Fentanyl in transdermalen therapeutischen Systemen in mehreren Ländern arzneimittelrechtlich zugelassen (vgl. K.A. Lehmann et al., Transdermal Fentanyl, Springer-Verlag, 1991). Aber auch andere Wirkstoffe mit Missbrauchspotential, wie z.B. Morphin und Buprenorphin, können transdermal verabreicht werden.

So offenbaren beispielsweise DE-A 39 39 376 und EP-A-1 323 421 ein transdermales therapeutisches System mit Buprenorphin als aktivem Bestandteil. EP-A 413 034 offenbart ein Laminat, welches eine Schicht aus einem für Feuchtigkeit undurchlässigen oder semipermeablen Material und zwei Klebeschichten aufweist, von denen zumindest eine einen Wirkstoff enthält. Als Wirkstoff kann das Laminat Buprenorphin Hydrochlorid enthalten. EP-A 484 543 offenbart ein Pflaster aus einer Filmschicht mit einer Schichtdicke von 0,5 bis 4,9 µm und einer Klebeschicht, die den Wirkstoff enthält. Als Wirkstoff kann das Pflaster Buprenorphin Hydrochlorid enthalten. EP-A 1 174 137 offenbart Buprenorphin enthaltende Pflaster, welche zur transkutanen Verabreichung des Wirkstoffs einen bestimmten Hilfsstoff enthalten.

Transdermale therapeutische Systeme, welche Buprenorphin oder andere starke Analgetika enthalten, werden beispielsweise zur Schmerztherapie bei Krebspatienten im fortgeschrittenen Stadium der Erkrankung eingesetzt, wodurch der Patient zu Hause bleiben und die Medikation selbst vornehmen kann. Das transdermale therapeutische System gewährleistet gegenüber anderen Darreichungsformen eine konstante Plasmakonzentration und eine geringe Gefahr der Überdosierung. Nachteilig ist jedoch, dass üblicherweise nach Gebrauch erhebliche Restmengen des Wirkstoffs im System zurückbleiben, häufig weit über 50% der ursprünglich enthaltenen Menge, teilweise 85% bis 90%, wodurch sich zusätzliche Probleme ergeben.

Starke Analgetika, wie Fentanyl, Morphin und Buprenorphin, sind nämlich neben der Schmerztherapie auch zum Missbrauch geeignet, so dass eine größere, nach Gebrauch im transdermalen therapeutischen System verbleibende Restmenge des Analgetikums eine potentielle Rauschmittelquelle für Personen darstellt, die einen Missbrauch beabsichtigen.

Infolge ihrer Abhängigkeit entwickeln diese Personen eine mitunter erstaunliche Kreativität bei der Beschaffung neuer Drogen. Um die Restmenge des Wirkstoffs aus dem transdermalen therapeutischen System zu extrahieren, benötigen sie üblicherweise nicht einmal besondere Hilfsmittel oder besonderen technischen Sachverstand. So gelingt die Isolierung des Wirkstoffs häufig bereits durch einfaches Herauslösen in reinem Ethanol, welches z.B. in Apotheken frei erhältlich ist.

Es besteht somit ein Bedarf an transdermalen therapeutischen Systemen, welche zur Verabreichung von Analgetika geeignet sind und die Nachteile der transdermalen therapeutischen Systeme des Standes der Technik überwinden.

Der Erfindung liegt die Aufgabe zugrunde, ein transdermales therapeutisches System zur Verabreichung von Analgetika bereitzustellen, welches Vorteile gegenüber den transdermalen therapeutischen Systemen des Standes der Technik bietet. Insbesondere sollte das transdermale therapeutische System die Möglichkeiten eines Missbrauchs des enthaltenen Wirkstoffs vermindern, ausgewogene Haftungseigenschaften und gute pharmakokinetische Parameter hinsichtlich der transdermalen Verabreichung des Analgetikums gewährleisten.

Es wurde überraschend gefunden, dass diese Aufgabe gelöst werden kann durch ein transdermales therapeutisches System zur Verabreichung eines Analgetikums umfassend
- eine Trägerschicht (T);
- eine Klebeschicht (K), welche unmittelbar an die Trägerschicht (T) angrenzt, auf einem druckempfindlichen Klebstoff basiert und das Analgetikum enthält; und
- ggf. eine abziehbare Schutzschicht (S);
wobei die Schichtdicke der Klebeschicht (K) weniger als 7,0 µm beträgt, und wobei die Klebeschicht (K) das Analgetikum in einer Menge von mehr als 0,60 g/m² enthält, bezogen auf die Fläche der Klebeschicht (K).

Überraschenderweise weist das erfindungsgemäße System ausgezeichnete Haftungseigenschaften auf, obwohl die Schichtdicke der Klebeschicht (K) weniger als 7,0 µm beträgt, vorzugsweise im Bereich von mehr als 2,0 µm und weniger als 5,0 µm liegt. Diese ausgezeichneten Haftungseigenschaften können sogar bei einer vergleichsweise hohen Konzentration des Analgetikums in der Klebeschicht (K) erreicht werden, vorzugsweise bei einer Konzentration des Analgetikums, welche nur geringfügig unterhalb seiner Sättigungskonzentration innerhalb der Klebeschicht (K) liegt.

Bei den Systemen des Standes der Technik wurde bisher davon ausgegangen, dass bei derart geringen Schichtdicken der Klebeschicht (K) keine ausreichende Haftung des Systems erreicht werden kann, insbesondere dann nicht, wenn gleichzeitig die Wirkstoffkonzentration in der Klebeschicht (K) hoch ist.

So offenbart beispielsweise EP-A 413 034, dass die Haftung eines Pflasters auf der Haut drastisch reduziert wird, wenn die Schichtdicke der Klebeschicht weniger als 15 µm beträgt. Ausweislich der Offenbarung wird es bei einer Schichtdicke von 10 µm oder darunter schwierig, überhaupt eine Haftung zu gewährleisten. Auch EP-A 484 543 offenbart, dass bei Reduzierung der Schichtdicke der Klebeschicht die Haftungseigenschaften verschlechtert werden.

Im Gegensatz dazu weist das erfindungsgemäße System, bei dem die Schichtdicke der Klebeschicht (K) unterhalb von 7,0 µm liegt, vorzugsweise oberhalb von 2,0 µm und unterhalb von 5,0 µm, ausgezeichnete Haftungseigenschaften auf, wobei gleichzeitig eine hohe Konzentration des Analgetikums in der Klebeschicht (K) ermöglicht wird.

Als Maß für die Haftungseigenschaften des erfindungsgemäßen Systems wird bevorzugt die Kraft gemessen, die erforderlich ist, um ein 4 cm breites System bei einer Geschwindigkeit von 50 mm/min orthogonal von einer ebenen Edelstahloberfläche abzuziehen. Üblicherweise werden dabei Werte gemessen, die innerhalb eines bestimmten Bereichs schwanken. Die mittlere Klebkraft kann als arithmetisches Mittel des gemessenen Maximalwertes und des gemessenen Minimalwertes angegeben werden. Erfindungsgemäß bevorzugt erfolgt die Messung wie in Beispiel 9 beschrieben. Bevorzugt beträgt die so bestimmte mittlere Klebkraft des erfindungsgemäßen Systems wenigstens 1,0 N/4cm, bevorzugter wenigstens 2,0 N/4cm, noch bevorzugter wenigstens 3,5 N/4cm, am bevorzugtesten wenigstens 5,0 N/4cm und insbesondere wenigstens 10 N/4cm.

Ferner kann mit Hilfe der erfindungsgemäßen Systeme überraschenderweise erreicht werden, dass innerhalb des bestimmungsgemäßen Zeitintervalls der Anwendung, üblicherweise innerhalb von 1 bis 5 Tagen, die weit überwiegende Menge des ursprünglich enthaltenen Analgetikums freigesetzt wird, so dass nach der Anwendung - wenn überhaupt - nur vergleichsweise geringe Rückstände im System verbleiben. Auf diese Weise wird der Missbrauch bereits verwendeter Systeme verhindert oder zumindest erheblich erschwert, da der Versuch der Extraktion des (restlichen) Wirkstoffs aus dem System praktisch erfolglos bleibt.

Das erfindungsgemäße transdermale therapeutische System zeichnet sich durch einen guten Tragekomfort aus. Hautreizungen während des Tragens werden vermieden und die mechanischen Eigenschaften gewährleisten eine ausreichende Beständigkeit gegen äußere Einflüsse. Tragekomfort und Beständigkeit gegen mechanische äußere Einflüsse sind aufeinander abgestimmt.

Die Schichtdicke der Klebeschicht (K) des erfindungsgemäßen Systems beträgt weniger als 7,0 µm, bevorzugter weniger als 6,5 µm, noch bevorzugter weniger als 6,0 µm, am bevorzugtesten weniger 5,5 µm und insbesondere weniger als 5,0 µm. Dabei beträgt die Schichtdicke der Klebeschicht (K) bevorzugt mehr als 2,0 µm. In einer bevorzugten Ausführungsform liegt die Schichtdicke der Klebeschicht (K) im Bereich von mehr als 2,0 µm und weniger als 7,0 µm. Bevorzugter liegt die Schichtdicke der Klebeschicht (K) im Bereich von 2,5 µm bis 6,5 µm, noch bevorzugter 2,5 µm bis 6,0 µm, am bevorzugtesten 2,5 µm bis 5,5 µm und insbesondere 2,5 µm bis weniger als 5,0 µm. Besonders bevorzugt liegt die Schichtdicke der Klebeschicht (K) im Bereich von mehr als 2,0 und weniger als 5,0 µm, insbesondere zwischen 2,5 und 4,5 µm.

Die Schichtdicke der Klebeschicht (K) kann mit Hilfe üblicher Methoden bestimmt werden. Beispielsweise kann die Schichtdicke der Klebeschicht (K) aus der Gesamtschichtdicke des Systems durch Subtraktion der Schichtdicke der Trägerschicht (T), der Schutzschicht (S) und ggf. weiterer neben der Klebeschicht (K) vorhandener Schichten berechnet werden, wobei die Gesamtschichtdicke des Systems und die Schichtdicke der einzelnen Schichten beispielsweise mit einer Mikrometerschraube oder mit einem Laser gemessen werden können.

Die erfindungsgemäßen Systeme zeichnen sich durch eine hohe Homogenität der Schichtdicke der Klebeschicht (K) aus. Da bei den erfindungsgemäßen Systemen das Analgetikum bevorzugt gleichmäßig in der Klebeschicht (K) verteilt ist, ist die Homogenität der Schichtdicke der Klebeschicht (K) üblicherweise proportional zur Homogenität der Verteilung des Analgetikums. Ist demnach an einer Stelle der Klebeschicht (K) weniger druckempfindlicher Klebstoff vorhanden, so ist an dieser Stelle auch entsprechend weniger Analgetikum vorhanden und die Homogenität der Verteilung des Analgetikums kann daher als Maß für die Homogenität der Schichtdicke der Klebeschicht (K) herangezogen werden.

Bevorzugt liegt die interindividuelle Variation der Schichtdicke der Klebeschicht (K) bei Vergleich zweier erfindungsgemäßer Systeme im Bereich von 100 ± 5,0 %, bevorzugter 100 ± 4,0 %, noch bevorzugter 100 ± 3,0 %, am bevorzugtesten 100 ± 2,0 % und insbesondere 100 ± 1,0 %.

Zur Bestimmung dieser interindividuellen Variation der Schichtdicke der Klebeschicht (K) kann das in den beiden Vergleichssystemen jeweils in der Klebeschicht (K) enthaltene Analgetikum quantifiziert werden, beispielsweise durch Extraktion mit Hilfe eines geeigneten Lösungsmittels und quantitative Analyse gemäß der in den einschlägigen Arzneibüchern für das betreffende Analgetikum angegebenen Methoden, z.B. UV-spektroskopisch. Als Bezugsfläche kann dabei die gesamte Klebeschicht (K) des erfindungsgemäßen Systems herangezogen werden, bevorzugt wird jedoch nur ein Teil der Fläche der Klebeschicht (K) untersucht und die Variation der Schichtdicke, bzw. die Variation der Menge des Analgetikums, auf diese Teilfläche bezogen. Bevorzugt beträgt die Bezugsfläche 10 cm², bevorzugter 5,0 cm² und noch bevorzugter 1,0 cm². Besonders bevorzugt beträgt bei den erfindungsgemäßen Systemen die Variation der Menge des Analgetikums bezogen auf eine Fläche von 1,0 cm² 100 ± 5,0 %, bevorzugter 100 ± 4,0 %, noch bevorzugter 100 ± 3,0 %, am bevorzugtesten 100 ± 2,0 % und insbesondere 100 ± 1,0 %.

Das erfindungsgemäße transdermale therapeutische System enthält als pharmazeutischen Wirkstoff ein Analgetikum. Neben diesem Analgetikum kann das erfindungsgemäße System weitere Wirkstoffe enthalten, z.B. weitere Analgetika, aber auch Wirkstoffe anderer Wirkstoffklassen. Bevorzugt enthält das erfindungsgemäße System nur ein Analgetikum als einzigen Wirkstoff.

Bevorzugt werden erfindungsgemäß als Analgetikum diejenigen pharmazeutischen Wirkstoffe verstanden, die von der WHO dem ATC-Index N02 zugeordnet werden (vorzugsweise in der amtlichen deutschen Fassung vom 1. Januar 2005). Bevorzugte Analgetika sind "Opioide" (ATC-Code N02A), "andere Analgetika und Antipyretika" (ATC-Code N02B) und "Migränemittel" (ATC-Code N02C). Innerhalb der bevorzugteren Opioide sind besonders bevorzugt die "natürlichen Opium-Alkaloide" (ATC-Code N02AA), "Phenylpiperidin-Derivate" (ATC-Code N02AB), "Diphenylpropylamin-Derivate" (ATC-Code N02AC), "Benzomorphan-Derivate" (ATC-Code N02AD), "Oripavin-Derivate" (ATC-Code N02AE), "Morphinan-Derivate" (ATC-Code N02AF), "Opioide in Kombination mit Spasmolytika" (ATC-Code N02AG) und "andere Opioide" (ATC-Code N02AX).

Somit enthält das erfindungsgemäße transdermale therapeutische System bevorzugt als Analgetikum ein Opioid, besonders bevorzugt ein Opioid ausgewählt aus der Gruppe bestehend aus Fentanyl, Alfentanil, Sufentanil, Phenoperidin, Anileridin, Remifentanil, Morphin, Diacetylmorphin, Hydromorphon, Nicomorphin, Oxycodon, Diamorphin, Ethylmorphin, Ketobemidon, Pethidin, Dextromoramid, Piritramid, Dextropropoxyphen, Bezitramid, Methadon, Pentazocin, Phenazocin, Buprenorphin, Butorphanol, Nalbufin, Tilidin, Dezocin, Meptazinol, (1 R,2R,4S)-2-[(Dimethylamino)methyl-4-(p-fluorbenzyloxy)-1-(m-methoxyphenyl)-cyclohexanol, (1 R,2R)-3-(2-Dimethylaminomethyl-cyclohexyl)-phenol, (1 R,2R)-3-(3-dimethylamino-1-ethyl-2-methyl-propyl)-phenol, (1S,2S)-3(3-Dimethylamino-1-ethyl-2-methyl-propyl)-phenol, (2R,3R)-1-Dimethylamino-3(3-methoxy-phenyl)-2-methyl-pentan-3-ol, (1 RS,3RS,6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)-phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure-3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyctohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride.

Insbesondere bevorzugt ist das Opioid Buprenorphin (ATC-Code N02AE01) als freie Base, in Form eines seiner pharmazeutisch verträglichen Salze, vorzugsweise Buprenorphin Hydrochlorid, Buprenorphin Saccharinat, Buprenorphin Formiat, Buprenorphin Mesylat, Buprenorphin Hydrogencitrat, Buprenorphin Nicotinat oder Buprenorphin Sebacinat, oder in Form eines seiner pharmazeutisch verträglichen Prodrugs. Erfindungsgemäß bevorzugte Prodrugs des Buprenorphins sind seine pharmazeutisch verträglichen Ester oder Ether.

Bevorzugt enthält das erfindungsgemäße therapeutische System das Analgetikum lediglich in der Klebeschicht (K), es ist jedoch grundsätzlich auch möglich, dass neben der Klebeschicht (K) weitere Schichten vorhanden sind, welche das Analgetikum oder einen anderen Wirkstoff enthalten. Bevorzugt enthält die Klebeschicht (K) das Analgetikum, vorzugsweise Buprenorphin, eines seiner pharmazeutisch verträglichen Salze oder Prodrugs, in einer Menge von 0,1 bis 25 Gew.-%, bevorzugter 1,0 bis 20 Gew.-%, noch bevorzugter 2,5 bis 17,5 Gew.-%, am bevorzugtesten 5,0 bis 15 Gew.-% und insbesondere 9,0 bis 12,5 Gew.-%, bezogen auf das Gesamtgewicht der Klebeschicht (K).

Besonders bevorzugt liegt die Konzentration des Analgetikums, vorzugsweise von Buprenorphin, eines seiner pharmazeutisch verträglichen Salze oder Prodrugs, innerhalb der getrockneten Klebeschicht (K) nur geringfügig unterhalb seiner Sättigungskonzentration. Die Sättigungskonzentration ist u.a. von der chemischen Natur des druckempfindlichen Klebstoffs abhängig und kann durch Routineversuche ermittelt werden.

Beispielsweise kann ein Kristall des Analgetikums auf die Oberfläche der zuvor hergestellten, ggf. getrockneten und bereits das Analgetikum enthaltenen Klebeschicht (K) aufgebracht werden. Nimmt die Klebeschicht (K) die als Kristall aufgebrachte Menge des Analgetikums noch auf, so liegt die Konzentration des Analgetikums in der Klebeschicht (K) noch unterhalb der Sättigungskonzentration. Führt hingegen das Aufbringen des Kristalls nicht zu dessen Aufnahme oder sogar zu einer Kristallisation des Analgetikums innerhalb der Klebeschicht (K), wirkt der Kristall gewissermaßen als Impfkristall, so liegt die Konzentration des Analgetikums bereits bei der Sättigungskonzentration. Liegt das Analgetikum in der Klebeschicht (K) teilweise kristallin vor, so ist die Sättigungskonzentration bereits überschritten.

Alternativ können in einer Versuchsreihe Klebeschichten (K) mit stetig ansteigender Konzentration des Analgetikums, beispielsweise in Schritten von 0,1 Gew.-% bezogen auf das Trockengewicht der Klebeschicht (K), hergestellt werden. Kann nach Fertigstellung der Klebeschicht (K), d.h. nach Trocknung und Lagerung über einen geeigneten Zeitraum, beispielsweise über 24 Stunden, eine Kristallisation zumindest eines Teils des darin enthaltenen Analgetikums beobachtet werden, so ist die Sättigungskonzentration bei dieser Probe bereits überschritten. Die Sättigungskonzentration ist bei derjenigen Probe in der entsprechenden Konzentration erreicht, bei der eine Kristallisation des Analgetikums gerade noch nicht zu beobachten ist.

Erfindungsgemäß bevorzugt liegt die Konzentration des Analgetikums in der getrockneten Klebeschicht (K) im Bereich von 90% bis 100% der oben genannten Sättigungskonzentration, bevorzugter 92,5% bis 100%, noch bevorzugter 95% bis 100% und insbesondere 97,5% bis 100%. Es wurde überraschend gefunden, dass eine ausgezeichnete Haftung der Klebeschicht (K) auch bei derart hoher Konzentration des Analgetikums in der Klebeschicht (K) erreicht werden kann, selbst wenn die Schichtdicke der Klebeschicht (K) unterhalb von 7 µm, vorzugsweise im Bereich von mehr als 2,0 µm und weniger als 5,0 µm liegt.

Bevorzugt enthält das erfindungsgemäße System den Wirkstoff in einer Menge, welche die Freisetzung von einer, zwei, drei, vier oder fünf Tagesdosen innerhalb von einem, zwei, drei, vier bzw. fünf Tagen gewährleistet. Besonders bevorzugt ist das erfindungsgemäße System für die Verwendung über 1 bis 5, bevorzugter 2 bis 4, insbesondere 3 Tage konfektioniert und enthält das Analgetikum in einer entsprechenden Menge, vorzugsweise Buprenorphin, eines seiner pharmazeutisch verträglichen Salze oder Prodrugs. Bei Buprenorphin beträgt die Freisetzung bevorzugt 35 bis 70 µg/h, was u.a. durch die Kontaktfläche des transdermalen therapeutischen Systems zur Haut reguliert werden kann.

Das erfindungsgemäße System hat vorzugsweise eine Gesamtfläche (Kontaktfläche zur Haut bei bestimmungsgemäßer Anwendung) von 10 bis 200 cm², bevorzugter 20 bis 150 cm².

Das erfindungsgemäße System enthält in der Klebeschicht (K) das Analgetikum, vorzugsweise Buprenorphin, eines seiner pharmazeutisch verträglichen Salze oder Prodrugs, in einer Menge von mehr als 0,60, 0,65, 0,70, 0,75, 0,80, 0,85, 0,90, 0,95, 1,00 oder 1,10 g/m². In einer anderen bevorzugten Ausführungsform enthält das erfindungsgemäße System in der Klebeschicht (K) das Analgetikum, vorzugsweise Buprenorphin, eines seiner pharmazeutisch verträglichen Salze oder Prodrugs, in einer Menge von mehr als 1,20, 1,30, 1,40, 1,50, 1,75, 2,0, 2,25, 2,5 oder 3,0 g/m², bezogen auf die Fläche der Klebeschicht (K). Experimentelle Untersuchungen deuten überraschenderweise darauf hin, dass bei den erfindungsgemäßen transdermalen therapeutischen Systemen der Rückstand des Analgetikums, welcher nach einer bestimmungsgemäßen Anwendung des transdermalen therapeutischen Systems darin zurückbleibt, relativ zur ursprünglich, d.h. vor der bestimmungsgemäßen Anwendung enthaltenen Menge des Analgetikums verringert werden kann, wenn die Flächenkonzentration des Analgetikums mehr als 0,60 g/m², vorzugsweise mehr als 0,80 g/m² beträgt. Diese Erkenntnis sollte jedoch nicht als Bindung an eine wissenschaftliche Theorie aufgefasst werden.

Bei den erfindungsgemäßen Systemen grenzt die Klebeschicht (K) unmittelbar an die Trägerschicht (T) an. Bevorzugt basiert die Trägerschicht (T) auf einem Polymer ausgewählt aus der Gruppe bestehend aus Polyolefinen, Olefin-Copolymerisaten, Polyestern, Co-Polyestern, Polyamiden, Co-Polyamiden, Polyurethanen, etc. Als Beispiele für geeignete Materialien können insbesondere genannt werden Polyester, bevorzugt Polyethylenterephthalate; Polyolefine, bevorzugt Polyethylene, Polypropylene oder Polybutylene; Polycarbonate; Polyethylenoxide; Polyurethane; Polystyrole; Polyamide; Polyimide; Polyvinylacetate; Polyvinylchloride; Polyvinylidenchloride; Copolymerisate, bevorzugt Acrylnitril-Butadien-Styrol-Terpolymere oder Ethylen-Vinylacetat-Copolymerisate. Besonders bevorzugt basiert die Trägerschicht (T) auf einem Polyester, insbesondere auf einem Polyethylenterephthalat, beispielsweise auf Hostaphan^{®} RN.

In einer bevorzugten Ausführungsform weist die Trägerschicht (T) des erfindungsgemäßen Systems eine Schichtdicke im Bereich von 5,0 bis 125 µm, bevorzugter 10 bis 115 µm noch bevorzugter 25 bis 100 µm, am bevorzugtesten 35 bis 95 µm und insbesondere 50 bis 85 µm auf.

In einer anderen bevorzugten Ausführungsform weist die Trägerschicht (T) des erfindungsgemäßen Systems eine Schichtdicke von mehr als 125 µm, vorzugsweise im Bereich von 125 bis 2.000 µm, bevorzugter von 130 bis 1.500 µm, noch bevorzugter von 140 bis 1.000 µm und insbesondere von 150 bis 500 µm auf. Es wurde gefunden, dass ein transdermales therapeutisches System bei zu geringer Gesamtschichtdicke durch flüchtiges Fühlen nicht ohne weiteres ertastet werden kann, was von einigen Patienten als unangenehm empfunden wird. Es kann daher von Vorteil sein, die Verringerung der Gesamtschichtdicke infolge der Herstellung extrem dünner Klebeschichten (K), wie es bei dem erfindungsgemäßen System der Fall ist, durch eine Erhöhung der Schichtdicke der Trägerschicht (T) zu kompensieren. Die Trägerschicht (T) kann dabei beispielsweise auch aus textilen Geweben oder geschäumten Materialien hergestellt sein, was dem Tragekomfort des erfindungsgemäßen Systems zuträglich sein kann. Ferner trägt eine vergleichsweise große Schichtdicke der Trägerschicht (T) zu einer besseren mechanischen Beständigkeit des erfindungsgemäßen Systems gegenüber äußeren Einflüssen bei.

Bevorzugt ist das Verhältnis der Schichtdicken der Trägerschicht (T) zur Klebeschicht (K) größer als 1,3:1, bevorzugter größer als 2,5:1, noch bevorzugter größer als 5,0:1, am bevorzugtesten größer als 7,5:1 und insbesondere größer als 10:1.

In einer bevorzugten Ausführungsform ist das Verhältnis der Schichtdicken der Trägerschicht (T) zur Klebeschicht (K) größer als 15:1, bevorzugter größer als 30:1, noch bevorzugter größer als 50:1, am bevorzugtesten größer als 75:1 und insbesondere größer als 100:1.

Hinsichtlich der Schichtdicke der Trägerschicht (T) und des Verhältnisses der Schichtdicke der Trägerschicht (T) zur Schichtdicke der Klebeschicht (K) [(T):(K)] sind erfindungsgemäß ferner folgende Kombinationen bevorzugt:

| | Trägerschicht (T) | (T):(K) |
|---|---|---|
| bevorzugt | 5,0 bis 125 µm | 2,5:1 bis 40:1 |
| bevorzugter | 10 bis 115 µm | 5,0:1 bis 35:1 |
| noch bevorzugter | 25 bis 100 µm | 7,5:1 bis 30:1 |
| am bevorzugtesten | 35 bis 95 µm | 10:1 bis 25:1 |
| insbesondere | 50 bis 85 µm | 12,5:1 bis 20:1 |

Bevorzugt bildet die Trägerschicht (T) eine der beiden Oberflächenschichten des erfindungsgemäßen Systems, d.h. ausgehend von der unmittelbar an die Trägerschicht (T) angrenzenden Klebeschicht (K) enthält das erfindungsgemäße transdermale therapeutische System jenseits der Trägerschicht (T) bevorzugt keine weitere Schicht.

In einer besonders bevorzugten Ausführungsform besteht das erfindungsgemäße transdermale therapeutische System aus der Trägerschicht (T), der Klebeschicht (K) und ggf. der abziehbaren Schutzschicht (S). Besonders bevorzugte Varianten dieser Ausführungsform enthalten als Analgetikum Buprenorphin, eines seiner pharmazeutisch verträglichen Salze oder Prodrugs und sind in nachfolgender Tabelle zusammengefasst:

| | bevorzugt | bevorzugter | insbesondere |
|---|---|---|---|
| Schichtdicke der Trägerschicht (T) | 5,0-125 µm | 25 - 100 µm | 50 - 85 µm |
| Schichtdicke der Klebeschicht (K) | mehr als 2,0 und weniger als 7,0 µm | mehr als 2,0 und weniger als 5,0 µm | 2,5 - 4,5 µm |
| Wirkstoffgehalt in der Klebeschicht (K) | 0,1 - 25 Gew.-% | 2,5 - 17,5 Gew.-% | 9,0 - 12,5 Gew.-% |

Weitere bevorzugte Varianten sind in nachfolgender Tabelle zusammengefasst:

| | bevorzugt | bevorzugter | insbesondere |
|---|---|---|---|
| Schichtdicke der Trägerschicht (T) | 125-2.000 µm | 140 - 1.000 µm | 150 - 500 µm |
| Schichtdicke der Klebeschicht (K) | mehr als 2,0 und weniger als 7,0 µm | mehr als 2,0 und weniger als 5,0 µm | 2,5 - 4,5 µm |
| Wirkstoffgehalt in der Klebeschicht (K) | 0,1 - 25 Gew.-% | 2,5 - 17,5 Gew.-% | 9,0 - 12,5 Gew.-% |

In einer besonders bevorzugten Ausführungsform ist das erfindungsgemäße System so beschaffen, dass nach Aufbringen der Klebeschicht (K) auf einer geeigneten Stelle der Haut eines Patienten innerhalb von 72 Stunden mehr als 20, 25 oder 30 Gew.-%, bevorzugter mehr als 35, 40 oder 45 Gew.-%, noch bevorzugter mehr als 50, 55 oder 60 Gew.-%, am bevorzugtesten mehr als 65, 70 oder 75 Gew.-% und insbesondere mehr als 80, 85, 90 oder 95 Gew.-% der ursprünglich in der Klebeschicht (K) enthaltenen Menge des Analgetikums, vorzugsweise Buprenorphin, eines seiner pharmazeutisch verträglichen Salze oder Prodrugs, freigesetzt werden.

Die Bestimmung der freigesetzten Wirkstoffmenge kann durch Routineversuche, ggf. mit Hilfe von Nacktratten, ermittelt werden. Beispielsweise kann das System nach Ablauf von 72 Stunden von der Haut entfernt werden und die im System enthaltene Restmenge extrahiert und gemäß der in den einschlägigen Arzneibüchern (z.B. US Pharmacopoeia, European Pharmacopoeia, Japanese Pharmacopoeia) für das jeweilige Analgetikum beschriebenen Verfahren quantifiziert werden. Handelt es sich bei dem Analgetikum um Buprenorphin, eines seiner pharmazeutisch verträglichen Salze oder Prodrugs, so kann bezüglich weiterer Einzelheiten beispielsweise verwiesen werden auf H.C. Evans, Drugs. 2003, 63(19):1999-2010; J. Sorge, Clin Ther. 2004, 26(11):1808-20; und R. Likar et al., Anesth Analg. 2005, 100(3):781-5.

Die Freisetzung von Buprenorphin kann alternativ *in vitro* mit Hilfe einer Franz-Diffusions-Zelle, vorzugsweise FDC-400, mit Hilfe von haarloser Mäusehaut bestimmt werden, beispielsweise wie beschrieben in H. Imoto et al., Biol Pharm Bull. 1996, 19(2):263-7 und in DE-A 102 37 056, auf deren Offenbarung vollumfänglich Bezug genommen wird. Für diesen Fall gelten als bevorzugte Ausführungsformen des erfindungsgemäßen Systems die vorstehend genannten Prozentangaben der Freisetzung nach 72 Stunden entsprechend. Einzelheiten hinsichtlich der Messung der transdermalen Permeation mit Hilfe einer Franz-Diffusions-Zelle sind u.a. beschrieben in T.J. Franz, J. lnvest. Dermatol. 64 , 190-195 (1975).

Die Klebeschicht (K) des erfindungsgemäßen Systems basiert auf einem druckempfindlichen Klebstoff. Druckempfindliche Klebstoffe (PSA) sind dem Fachmann bekannt. In diesem Zusammenhang kann beispielsweise verwiesen werden auf I. Benedek, Pressure-Sensitive Adhesives and Applications, Marcel Dekker, 2nd edition, 2004. Bevorzugt enthält der druckempfindliche Klebstoff, auf welchem die Klebeschicht (K) basiert, ein Polymer oder mehrere Polymere ausgewählt aus der Gruppe bestehend aus Polyacrylaten, Polyvinylethern, Polyvinylalkohol, Polyisobutylenen, Acrylat-Copolymerisaten, Ethylen-Vinylacetat-Copolymerisaten, Styrol-Isopren-Copolymerisaten, Styrol-Butadien-Copolymerisaten, Silikonen und hydrierten Estern des Kolophoniums. Besonders bevorzugt sind Acrylat-Vinylacetat-Copolymerisate, welche beispielsweise unter der Bezeichnung DURO-TAK^{®} im Handel erhältlich sind. Besonders bevorzugt werden diese in Kombination mit Polyacrylaten, beispielsweise Carbopol^{®}, insbesondere Carbopol^{®} 980, oder mit Hydroxypropylcellulose als Viskositätsverbesserer eingesetzt.

Zur Verbesserung der Permeation des in dem erfindungsgemäßen System enthaltenen Analgetikums, vorzugsweise Buprenorphin, eines seiner pharmazeutisch verträglichen Salze oder Prodrugs, enthält das erfindungsgemäße System bevorzugt einen oder mehrere Hilfsstoffe. In diesem Zusammenhang kann beispielsweise verwiesen werden auf D.S. Hsieh, Drug Permeation Enhancement: Theory and Applications (Drugs and the Pharmaceutical Sciences: a Series of Textbooks and Monographs), Marcel Dekker, 1994 und E.W. Smith et al., Percutaneous Penetration Enhancers, CRC Press, 1995.

Vorzugsweise enthält die Klebeschicht (K) zumindest einen Hilfsstoff zur Verbesserung der transdermalen Permeation des Analgetikums, vorzugsweise von Buprenorphin, von einem seiner pharmazeutisch verträglichen Salze oder Prodrugs, wobei der Hilfsstoff ausgewählt ist aus der Gruppe bestehend aus Tensiden {z.B. nichtionischen Tensiden, amphoterischen Tensiden, anionischen Tensiden, kationischen Tensiden, etc, einschließlich Fettsäureester, Fettalkohole, Polyoxyethylen-gehärtetes Ricinusöl (HCO), wie HCO-10, HCO-40, HCO-50, HCO-60; Polysolvat (Tween^{®}), wie z.B. Tween^{®}-60, Tween^{®}-65, Tween^{®}-80; Sorbitanester, wie Sorbitantrioleat, Sorbitanmonopalmitat, Sorbitanmonolaurat, Sorbitansesquioleat, Polyoxyethylen/Polyoxypropylen-Sorbitan-mono-Fettsäureester, Sorbitan-Polyoxyethylen(160)-polyoxypropylen(30)-glykol-monostearat; Glycerinester wie Glycerinmonostearat, Triacetin; Benzalkoniumchlorid; Benzetoniumchlorid}; Aminen {z.B. Monoethanolamin, Diethanolymin, Triethanolamin, Diisopropanolamin, Triisopropanolamin}; anorganischen Basen {z.B. NaOH, KOH, Ca(OH)₂, NaHCO₃}; Polyvinylpyrrolidon; Propylenglykol; Benzylalkohol; Menthol; Isosorbidnitrat; Dodecylazacycloheptan-2-on; Milchsäure; und Ethanol.

In einer anderen bevorzugten Ausführungsform enthält das erfindungsgemäße System in der Klebeschicht (K) keinen Hilfsstoff zur Verbesserung der transdermalen Permeation des Analgetikums, vorzugsweise überhaupt keinen Hilfsstoff.

In einer bevorzugten Ausführungsform enthält das erfindungsgemäße transdermale therapeutische System eine abziehbare Schutzschicht (S). Diese ist bevorzugt auf zumindest der Oberfläche, welche der Klebeschicht (K) zugewandt ist, silanisiert' und/oder fluoridiert, und wird vor der Anwendung des Systems entfernt. Dadurch wird die Oberfläche der Klebeschicht (K) freigelegt. Bevorzugt grenzt die Schutzschicht (S) unmittelbar an die Klebeschicht (K) an.

Geeignete Materialien, auf denen die Schutzschicht (S) basieren kann, sind dem Fachmann bekannt. Bevorzugt basiert die Schutzschicht (S) auf wenigstens einem Material ausgewählt aus der Gruppe bestehend aus Polyester, Polypropylen, Polyvinylchlorid, Aluminium und Papier, wobei zumindest die an die Klebeschicht (K) angrenzende Seite bevorzugt eine Silikon- und/oder Polyethylen- und/oder Fluorsilikon- und/oder Polytetrafluorethylen-Beschichtung aufweist. Bevorzugt basiert die Schutzschicht (S) auf einem Polyester, beispielsweise auf Hostaphan^{®} RNT. Bevorzugt weist die Schutzschicht (S) eine Dicke von 10 bis 100 µm, bevorzugter von 25 bis 50 µm auf.

Das erfindungsgemäße transdermale therapeutische System ist vorzugsweise als wirkstoffhaltiges Pflaster ausgebildet, wobei die Wirkstofffreisetzung matrixgesteuert ist.

Das erfindungsgemäße System kann weitere übliche Hilfs- und Zusatzstoffe enthalten, beispielsweise lipophile Zusatzstoffe wie z.B. fettlösliche Vitamine, ungesättigte Fettsäuren, Campher, Nachtkerzenöl, Borretschöl, Johannisbeerkernöl, Fischöle, Lebertran, Ceramide, aber auch Farbstoffe, Weichmacher, Lösungsmittel, hautglättende Mittel und Duftstoffe. In diesem Zusammenhang kann beispielsweise verwiesen werden auf H.P. Fiedler, Lexikon der Hilfsstoffe für Pharmazie, Kosmetik und angrenzende Gebiete, Editio Cantor Aulendorff, 2002.

Ein weiterer Aspekt der Erfindung betrifft ein Verfahren zur Herstellung eines vorstehend beschriebenen transdermalen therapeutischen Systems umfassend die Schritte
(a) Mischen der Inhaltsstoffe der Klebeschicht (K) ggf. in einem geeigneten Lösungsmittel;
(b) Auftragen der in Schritt (a) erhaltenen Mischung auf eine der beiden Oberflächen der Trägerschicht (T);
(c) ggf. Trocknen der in Schritt (b) aufgetragenen Mischung; und
(d) ggf. Aufbringen der Schutzschicht (S) auf die in Schritt (b) aufgetragene und ggf. in Schritt (c) getrocknete Mischung.

Es wurde überraschend gefunden, dass sich mit Hilfe des erfindungsgemäßen Verfahrens vergleichsweise dünne Klebeschichten (K) mit vergleichsweise hoher Konzentration des darin befindlichen Analgetikums herstellen lassen, wobei gute Haftungseigenschaften erzielt werden können. Ferner weist die Schichtdicke der Klebeschicht (K) der nach dem erfindungsgemäßen Verfahren hergestellten Systeme eine ausgezeichnete Homogenität auf.

In einer bevorzugten Ausführungsform des erfindungsgemäßen Verfahrens wird in Schritt (b) die Mischung mit Hilfe eines Filmziehgeräts aufgetragen, wobei die Spaltbreite der Rakel im Bereich von 10 bis 50 µm, bevorzugter 15 bis 45 µm, noch bevorzugter 20 bis 40 µm, am bevorzugtesten 25 bis 35 µm liegt und insbesondere die Spaltbreite der Rakel 30 µm beträgt.

Geeignete Filmziehgeräte sind dem Fachmann bekannt. Beispielsweise eignet sich ein Erichsen Filmziehgerät Coatmaster 509/MC-1.

Beim erfindungsgemäßen Verfahren liegt in Schritt (b) die Auftragungsgeschwindigkeit der Mischung bevorzugt im Bereich von 1,0 bis 25 mm/s, bevorzugter 2,0 bis 10 mm/s, noch bevorzugter 3,0 bis 7,0 mm/s, am bevorzugtesten 4,0 bis 6,0 mm/s und insbesondere beträgt die Auftragungsgeschwindigkeit 5,0 mm/s.

Besonders bevorzugt wird die Auftragungsgeschwindigkeit so gewählt, dass das Verhältnis der Spaltbreite der Rakel zur Schichtdicke der in Schritt (b) aufgetragenen Mischung, ggf. nach Trocknen der Mischung in Schritt (c), zumindest 1:1, bevorzugter zumindest 2:1, noch bevorzugter zumindest 3:1, am bevorzugtesten zumindest 4:1 und insbesondere zumindest 5:1 beträgt. Bei einer Spaltbreite von 30 µm kann dies beispielsweise durch eine Auftragungsgeschwindigkeit von etwa 5 mm/s erreicht werden. Eine genaue Einstellung ist durch einfache Routineversuche möglich.

Das vorstehend definierte Verhältnis aus Spaltbreite und Schichtdicke der - ggf. getrockneten - in Schritt (b) aufgetragenen Schicht (Klebeschicht (K)) wird erfindungsgemäß auch als "Kollabierfaktor" bezeichnet. Es wurde überraschend gefunden, dass besonders vorteilhafte transdermale therapeutische Systeme erhältlich sind, wenn der Kollabierfaktor einen Wert von 2 oder darüber, bevorzugter 3 oder darüber, noch bevorzugter 4 oder darüber und insbesondere 5 oder darüber aufweist. In diesem Fall werden ausgezeichnete Haftungseigenschaften bei gleichzeitig nur sehr geringer Schichtdicke der Klebeschicht (K) erreicht, auch wenn das darin enthaltene Analgetikum in hoher Konzentration vorliegt, beispielsweise nur geringfügig unterhalb seiner Sättigungskonzentration.

Bevorzugt weist die in Schritt (a) hergestellte Mischung eine Viskosität (bestimmt mit einem Brookfield-Viskosimeter bei 25°C, #27, 20 Upm, SSA, EtOAc/Heptan 87/13) im Bereich von 1.000 bis 10.000 mPa·s, bevorzugter von 2.000 bis 7.500 mPa·s und noch bevorzugter von 2.500 bis 4.500 mPa·s auf.

Bevorzugt enthält die in Schritt (a) hergestellte Mischung ein Lösungsmittel. Besonders bevorzugt handelt es sich dabei um ein organisches Lösungsmittel, welches bei Atmosphärendruck einen Siedepunkt von weniger als 100°C, bevorzugter weniger als 90°C, noch bevorzugter weniger als 80°C und am bevorzugtesten weniger als 70 °C aufweist. Ethanol ist besonders bevorzugt.

Der ggf. durchgeführte Trocknungsschritt (c) des erfindungsgemäßen Verfahrens kann bei erhöhter Temperatur und/oder vermindertem Druck durchgeführt werden. Bevorzugt wird er unter solchen Bedingungen und für eine Dauer durchgeführt, dass der Restanteil des verwendeten Lösungsmittels in der Klebeschicht (K) nach der Trocknung unterhalb von 1,0 Gew.-%, bevorzugter unterhalb von 0,5 Gew.-%, noch bevorzugter unterhalb von 0,1 Gew.-%, am bevorzugtesten unterhalb von 0,05 Gew.-% und insbesondere unterhalb von 0,01 Gew.-% liegt.

Der vorstehend genannte Kollabierfaktor wird insbesondere durch folgende Faktoren beeinflusst: die Art und Menge des verwendeten Lösungsmittels, die Auftragungsgeschwindigkeit, die Trocknungsbedingungen und die Viskosität der aufgetragenen Mischung. Durch einfache Routineversuche kann ermittelt werden, auf welche Werte diese Parameter einzustellen sind, um einen bestimmten Kollabierfaktor zu erzielen.

Es wurde überraschend gefunden, dass das erfindungsgemäße Verfahren zur Herstellung von transdermalen therapeutischen Systemen Vorteile gegenüber den herkömmlichen Verfahren des Standes der Technik hat, welche insbesondere bei der Herstellung von extrem dünnen Klebeschichten (K) zum Ausdruck kommen. So ist es bei herkömmlichen Verfahren zur Erzeugung dünner Klebeschichten (K) mitunter erforderlich, den Abstand zweier paralleler Walzen, durch welche eine bis zu mehrere Meter breite Folienbahn geführt wird, auf einen Abstand einzustellen, welcher in der Größenordnung weniger µm liegt. Dies stellt besonders hohe Anforderungen an die Oberflächenbeschaffenheit der beiden Walzen und die Justierung der Lager. So führen bereits kleinste Abweichungen bei Schichtdicken der Klebeschicht (K) in der Größenordnung von weniger als 7 µm zu erheblichen relativen Schwankungen der Schichtdicke der Klebeschicht (K) über die Breite der Folienbahn quer zu ihrer Laufrichtung.

Demgegenüber hat das erfindungsgemäße Verfahren den Vorteil, dass keine derartigen Walzen erforderlich sind, sondern die Zusammensetzung, welche später die Klebeschicht (K) bildet, z.B. mit Hilfe eines Filmziehgeräts auf eine planare Oberfläche aufgetragen werden kann und sich bereits infolge der gleichmäßig wirkenden Gravitationskraft eine sehr gleichmäßige Schichtdicke einstellt. Der Trocknungsvorgang erfolgt ebenfalls über die gesamte Fläche gleichmäßig, so dass letztlich eine sehr dünne Klebeschicht (K) mit allenfalls sehr geringen Schwankungen der Schichtdicke erhalten werden kann.

Die Erfindung betrifft auch transdermale therapeutische Systeme, welche nach dem vorstehend beschriebenen Verfahren erhältlich sind.

Ein weiterer Aspekt der Erfindung betrifft die Verwendung eines Analgetikums, vorzugsweise von Buprenorphin, eines seiner pharmazeutisch verträglichen Salze oder Prodrugs, zur Herstellung eines vorstehend beschriebenen transdermalen therapeutischen Systems zur Behandlung von Schmerz. Bevorzugt ist das erfindungsgemäße System dabei zur Anwendung über 1 bis 5, bevorzugter 2 bis 4, insbesondere 3 oder 4 Tage konfektioniert. In einer bevorzugten Ausführungsform ist der Schmerz ausgewählt aus der Gruppe bestehend aus akuten Schmerzen; chronischen Schmerzen; neuropathischen Schmerzen; visceralen Schmerzen; nocizeptiven Schmerzen; postoperativen Schmerzen; Phantomschmerzen; Kopfschmerzen, insbesondere Migräne-Kopfschmerzen, Clusterkopfschmerzen, Herpes-Neuralgie, Spannungskopfschmerzen, paroxysmale Hemikranie; Gesichtsschmerzen, insbesondere Trigeminusneuralgie; Zahnschmerzen; Kieferschmerzen; Wehenschmerzen; Geburtsschmerzen; Entbindungsschmerzen; Schmerzen bei Cystitis; Knochenschmerzen; Rückenschmerzen; Kreuzschmerzen; Gelenkschmerzen; Verbrennungsschmerzen; Herzschmerzen; Anginaschmerzen; muskulären Schmerzen, insbesondere Fibromyalgie; Bauchschmerzen; Magenschmerzen; und Krebsschmerzen.

In einer bevorzugten Ausführungsform ist der Schmerz Krebsschmerz ausgewählt aus der Gruppe bestehend aus Schmerz bei Hirntumoren, Knochenkrebs, Mundkrebs, Rachenkrebs, Speiseröhrenkrebs, Magenkrebs, Leberkrebs, Blasenkrebs, Bauchspeicheldrüsenkrebs, Krebs der Eierstöcke, Gebärmutterhalskrebs, Brustkrebs, Lungenkrebs, Hautkrebs, Prostatakrebs, Adenokarzinom, Basalzellenkarzinom, Krebs im Gastrointestinalbereich, Hautkrebs und Nierenkrebs.

Die nachfolgenden Beispiele dienen zur näheren Erläuterung der Erfindung, sind jedoch nicht einschränkend hinsichtlich ihres Umfangs auszulegen:

### Beispiel 1:

Mittels eines Erichsen Filmziehgeräts "Coatmaster 509/MC-1" wurde aus 20 g DURO-TAK^{®} 387-2510 mit einem Feststoffanteil von 41% eine Schicht von 30 µm auf eine Hostaphanfolie RN 75 aufgezogen. Die Auftragungsgeschwindigkeit betrug 5 mm/s. Nach einer Trocknungszeit von 2 Stunden wurde die gesamte Fläche der getrockneten Klebeschicht mit einer Hostaphanfolie RNT 36 (einseitig silikonisiert) als Schutzschicht abgedeckt.

Die Schichtdicke der eingesetzten Hostaphanfolien RN 75 und RNT 36 wurde mit Hilfe einer Mikrometerschraube auf 79 µm bzw. 38 µm bestimmt. Die Gesamtschichtdicke des Verbundes betrug 121 µm (Bestimmung mit Hilfe einer Mikrometerschraube), die Schichtdicke der Klebeschicht 4 µm.

### Beispiel 2 (Veraleich):

Wie in Beispiel 1 wurde mittels eines Erichsen Filmziehgeräts "Coatmaster 509/MC-1" aus 20 g DURO-TAK^{®} 387-2510 mit einem Feststoffanteil von 41% eine Schicht von 30 µm auf eine Hostaphanfolie RN 75 aufgezogen. Die Auftragungsgeschwindigkeit betrug hier jedoch 70 mm/s.

Nach einer Trocknungszeit von 2 Stunden wurde die gesamte Fläche der getrockneten Klebeschicht mit einer Hostaphanfolie RNT 36 (einseitig silikonisiert) als Schutzschicht abgedeckt.

Die Schichtdicke der eingesetzten Hostaphanfolien RN 75 und RNT 36 wurde mit Hilfe einer Mikrometerschraube auf insgesamt 114 µm bestimmt. Die Gesamtschichtdicke des Verbundes betrug 160 µm, die Schichtdicke der Klebeschicht 46 µm.

### Beispiel 3:

Analog zu Beispiel 1 wurde aus 16 g DURO-TAK® 387-2510 mit einem Feststoffanteil von 41% und 4 g Ethanol ein Verbund hergestellt. Die Schichtdicke der eingesetzten Hostaphanfolien RN 75 und RNT 36 betrug insgesamt 118 µm. Nach dem Trocknen betrug die Gesamtschichtdicke des Verbunds 122 µm und die Schichtdicke der Klebeschicht 4 µm.

### Beispiel 4:

Analog zu Beispiel 1 wurde aus 16 g DURO-TAK^{®} 387-2510 mit einem Feststoffanteil von 41% und 4 g 2%iges Carbopol 980/Ethanol-Gel (enthaltend 0,08 g Carbopol 980) ein Verbund hergestellt. Die Schichtdicke der eingesetzten Hostaphanfolien RN 75 und RNT 36 betrug insgesamt 116 µm. Nach dem Trocknen betrug die Gesamtschichtdicke des Verbunds 121 µm und die Schichtdicke der Klebeschicht 5 µm.

### Beispiel 5:

Analog zu Beispiel 1 wurde aus 16 g DURO-TAK^{®} 387-2510 mit einem Feststoffanteil von 41 % und 4 g 2%iges Hydroxypropylcellulose/Ethanol-Gel (enthaltend 0,08 g Hydroxypropylcellulose) ein Verbund hergestellt. Die Schichtdicke der eingesetzten Hostaphanfolien RN 75 und RNT 36 betrug insgesamt 116 µm. Nach dem Trocknen betrug die Gesamtschichtdicke des Verbunds 122 µm und die Schichtdicke der Klebeschicht 6 µm.

### Beispiel 6:

Analog zu Beispiel 1 wurde aus 10 g DURO-TAK^{®} 387-2510 mit einem Feststoffanteil von 41% und 10 g 2%iges Hydroxypropylcellulose/Ethanol-Gel (enthaltend 0,2 g Hydroxypropylcellulose) ein Verbund hergestellt. Die Schichtdicke der eingesetzten Hostaphanfolien RN 75 und RNT 36 betrug insgesamt 112 µm. Nach dem Trocknen betrug die Gesamtschichtdicke des Verbunds 116 µm und die Schichtdicke der Klebeschicht 4 µm.

### Beispiel 7:

Analog zu Beispiel 1 wurde aus 10 g DURO-TAK^{®} 387-2510 mit einem Feststoffanteil von 41% und 10 g 2%iges Carbopol 980/Ethanol-Gel (enthaltend 0,2 g Carbopol 980) ein Verbund hergestellt. Die Schichtdicke der eingesetzten Hostaphanfolien RN 75 und RNT 36 betrug insgesamt 112 µm. Nach dem Trocknen betrug die Gesamtschichtdicke des Verbunds 116 µm und die Schichtdicke der Klebeschicht 4 µm.

### Beispiel 8:

Die Klebkraft der in Beispielen 1, 3, 4, 5, 6 und 7 hergestellten Pflaster wurde mit Hilfe einer Materialprüfmaschine TestControl, Typ BTC FR2.5TH.D09 der Fa *Zwick*/*Roell* gemessen. Dazu wurde ein 4 cm breites Stück Pflaster längs zu 75% auf die obere Edelstahlplatte eines fahrbaren Schlittens aufgebracht. Der Schlitten wurde unterhalb des Messwertaufnehmers positioniert und befestigt. Das nicht verklebte

Reststück (25%) wurde so in die Pneumatikbacken des Messgerätes eingespannt, dass das Pflaster senkrecht nach oben zeigte. Nun wurde das Pflaster bei Raumtemperatur mit einer Geschwindigkeit von 50 mm/min nach oben vom Schlitten abgezogen. Beim Abziehen wurde der Schlitten automatisch nachgeführt, so dass das Pflaster stets senkrecht abgezogen wurde. Die Kräfte wurden in Abhängigkeit vom Weg aufgezeichnet.

Die Messergebnisse sind in folgender Tabelle zusammengefasst:

| Bsp.: | Schichtdicke Klebeschicht (K) (nach Trocknen) | DURO-TAK® 387-2510 | weitere Bestandteile der Mischung | Abzugskraft in N/4cm | |
|---|---|---|---|---|---|
| | | | | gemessen | Mittelwert |
| 1 | 6 µm | 20 g | - | 4,0-6,0 | 5,0 |
| 3 | 4 µm | 16g | 4 g EtOH | 1,6 - 3,0 | 2,3 |
| 4 | 5 µm | 16g | 4 g 2%iges Carbopol 980/EtOH-Gel | 14-24 | 19 |
| 5 | 6 µm | 16g | 4 g 2%iges H PC / EtOH-Gel | 1,5 - 3,0 | 2,3 |
| 6 | 4 µm | 10g | 10 g 2%iges HPC / EtOH-Gel | 1,2 - 2,1 | 1,7 |
| 7 | 4 µm | 10g | 10 g 2%iges Carbopol 980/EtOH-Gel | 0,6 - 1,3 | 1,0 |

## Patentansprüche

1. Transdermales therapeutisches System zur Verabreichung eines Analgetikums umfassend
- eine Trägerschicht (T);
- eine Klebeschicht (K), welche unmittelbar an die Trägerschicht (T) angrenzt, auf einem druckempfindlichen Klebstoff basiert und das Analgetikum enthält; und
- ggf. eine abziehbare Schutzschicht (S);
wobei die Schichtdicke der Klebeschicht (K) weniger als 7,0 µm beträgt und wobei die Klebeschicht (K) das Analgetikum in einer Menge von mehr als 0,60 g/m² enthält, bezogen auf die Fläche der Klebeschicht (K).

2. System nach Anspruch 1, **dadurch gekennzeichnet, dass** die Klebeschicht (K) das Analgetikum in einer Menge von mehr als 0,80 g/m² enthält, bezogen auf die Fläche der Klebeschicht (K).

3. System nach Anspruch 1 oder 2, **dadurch gekennzeichnet, dass** nach Aufbringen der Klebeschicht (K) auf eine geeignete Stelle der Haut eines Patienten innerhalb von 72 Stunden mindestens 20 Gew.-%, vorzugsweise mindestens 50 Gew.-%, der ursprünglich in der Klebeschicht (K) enthaltenen Menge des Analgetikums freigesetzt werden.

4. System nach einem der vorstehenden Ansprüche, **dadurch gekennzeichnet, dass** die Klebeschicht (K) das Analgetikum in einer Menge von 0,1 bis 25 Gew.-% enthält, bezogen auf das Gesamtgewicht der Klebeschicht (K).

5. System nach einem der vorstehenden Ansprüche, **dadurch gekennzeichnet, dass** die Konzentration des Analgetikums innerhalb der Klebeschicht (K) im Bereich von 90% bis 100% seiner Sättigungskonzentration liegt.

6. System nach einem der vorstehenden Ansprüche, **dadurch gekennzeichnet, dass** die Schichtdicke der Klebeschicht (K) größer als 2,0 µm und kleiner als 5,0 µm ist.

7. System nach einem der vorstehenden Ansprüche, **dadurch gekennzeichnet, dass** das Analgetikum ein Opioid ist.

8. System nach Anspruch 7, **dadurch gekennzeichnet, dass** das Opioid ausgewählt ist aus der Gruppe bestehend aus Fentanyl, Alfentanil, Sufentanil, Phenoperidin, Anileridin, Remifentanil, Morphin, Diacetylmoiphin, Hydromorphon, Nicomorphin, Oxycodon, Diamorphin, Ethylmorphin, Ketobemidon, Pethidin, Dextromoramid, Piritramid, Dextropropoxyphen, Bezitramid, Methadon, Pentazocin, Phenazocin, Buprenorphin, Butorphanol, Nalbufin, Tilidin, Dezocin, Meptazinol, (1R,2R)-3-(3-Dimethylamino-1-ethyl-2-methyl-propyl)-phenol, (2R,3R)-1-Dimethylamino-3-(3-methoxy-phenyl)-2-methyl-pentan-3-ol, 6-Dimethylaminomethyl-1-(3-methoxyphenyl)-cyclohexan-1,3-diol, (1R,2R)-3-(2-Dimethylaminomethyl-cyclohexyl)-phenol und deren physiologisch verträglichen Salzen bzw. Prodrugs.

9. System nach Anspruch 8, **dadurch gekennzeichnet, dass** das Opioid Buprenorphin, eines seiner pharmazeutisch verträglichen Salze oder Prodrugs ist.

10. System nach einem der vorstehenden Ansprüche, **dadurch gekennzeichnet, dass** die mittlere Kraft, die erforderlich ist, um ein 4 cm breites System bei einer Geschwindigkeit von 50 mm/min orthogonal von einer ebenen Edelstahloberfläche abzuziehen, wenigstens 1,0 N/4cm beträgt, vorzugsweise wenigstens 2,0 N/4cm und insbesondere wenigstens 3,5 N/4cm.

11. System nach einem der vorstehenden Ansprüche, **dadurch gekennzeichnet, dass** die Trägerschicht (T) eine Schichtdicke im Bereich von 5,0 bis 125 µm oder von 125 bis 2.000 µm aufweist.

12. System nach einem der vorstehenden Ansprüche, **dadurch gekennzeichnet, dass** die Trägerschicht (T) eine der beiden Oberflächenschichten des Systems bildet.

13. System nach einem der vorstehenden Ansprüche, **dadurch gekennzeichnet, dass** der druckempfindliche Klebstoff, auf welchem die Klebeschicht (K) basiert, ein Polymer enthält ausgewählt aus der Gruppe bestehend aus Polyacrylaten, Polyisobutylen und Silikonen.

14. System nach einem der vorstehenden Ansprüche, **dadurch gekennzeichnet, dass** die Klebeschicht (K) zumindest einen Hilfsstoff zur Verbesserung der transdermalen Permeation des Analgetikums enthält, wobei der Hilfsstoff ausgewählt ist aus der Gruppe bestehend aus Tensiden, Aminen, anorganischen Basen, polyvinylpyrrolidon, Propylenglykol, Benzylalkohol, Menthol, Isosorbidnitrat, Dodecylazacycloheptan-2-on, Milchsäure und Ethanol.

15. Verfahren zur Herstellung eines transdermalen therapeutischen Systems nach einem der vorstehenden Ansprüche umfassend die Schritte
(a) Mischen der Inhaltsstoffe der Klebeschicht (K), ggf. in einem geeigneten Lösungsmittel;
(b) Auftragen der in Schritt (a) erhaltenen Mischung auf eine der beiden Oberflächen der Trägerschicht (T);
(c) ggf. Trocknen der in Schritt (b) aufgetragenen Mischung; und
(d) ggf. Aufbringen der Schutzschicht (S) auf die in Schritt (b) aufgetragene und ggf. in Schritt (c) getrocknete Mischung.

16. Verfahren nach Anspruch 15, **dadurch gekennzeichnet, dass** in Schritt (b) die Mischung mit Hilfe eines Filmziehgeräts aufgetragen wird, wobei die Spaltbreite der Rakel im Bereich von 10 bis 50 µm liegt.

17. Verfahren nach Anspruch 16, **dadurch gekennzeichnet, dass** in Schritt (b) die Auftragungsgeschwindigkeit im Bereich von 1,0 bis 25 mm/s liegt.

18. Verfahren nach Anspruch 17, **dadurch gekennzeichnet, dass** die Auftragungsgeschwindigkeit so gewählt wird, dass das Verhältnis der Spaltbreite der Rakel zur Schichtdicke der in Schritt (b) aufgetragenen Mischung, ggf. nach Trocknen der Mischung in Schritt (c), zumindest 1:1 beträgt.

19. Verfahren nach einem der Ansprüche 15 bis 18, **dadurch gekennzeichnet, dass** die in Schritt (a) hergestellte Mischung eine Viskosität im Bereich von 1.000 bis 10.000 mPa·s aufweist.

20. Verfahren nach einem der Ansprüche 15 bis 19, **dadurch gekennzeichnet, dass** die in Schritt (a) hergestellte Mischung ein organisches Lösungsmittel enthält, welches bei Atmosphärendruck einen Siedepunkt von weniger als 100°C aufweist.

## Claims

1. A transdermal therapeutic system for administration of an analgesic, comprising
- a carrier layer (T):
- an adhesive layer (K) that immediately adjoins the carrier layer (T), is based on a pressure-sensitive adhesive, and contains the analgesic, and
- optionally, a strippable protective layer (S), the layer thickness of said adhesive layer (K) being less than 7.0 µm and the content of said analgesic in said adhesive layer (K) being greater than 0.60 g/m², based on the area of said adhesive layer (K).

2. The system according to claim 1, **characterized in that** the content of said analgesic in said adhesive layer (K) is greater than 0.80 g/m², based on the area of said adhesive layer (K).

3. The system according to claim 1 or claim 2, **characterized in that** after application of said adhesive layer (K) to a suitable part of the skin of a patient at least 20 wt%, preferably at least 50 wt%, of the amount of analgesic originally present in said adhesive layer (K) is released within a period of 72 hours.

4. The system according to any one of the previous claims, **characterized in that** said adhesive layer (K) contains said analgesic in a concentration of from 0.1 to 25 wt%, based on the total weight of said adhesive layer (K).

5. The system according to any one of the previous claims, **characterized in that** the concentration of said analgesic in said adhesive layer (K) ranges from 90 % to 100 % of its saturation point.

6. The system according to any one of the previous claims, **characterized in that** the layer thickness of said adhesive layer (K) is greater than 2.0 µm and less than 5.0 µm.

7. The system according to any one of the previous claims, **characterized in that** said analgesic is an opioid.

8. The system according to claim 7, **characterized in that** said opioid is selected of the group consisting of fentanyl, alfentanil, sufentanil, phenoperidine, anileridine, remifentanil, morphine, diacetylmorphine, hydromorphone, nicomorphine, oxycodone, diamorphine, ethylmorphine, ketobemidone, pethidine, dextromoramide, piritramide, dextropropoxyphene, bezitramide, methadone, pentazocine, phenazocine, buprenorphine, butorphanol, nalbufine, tilidine, dezocine, meptazinol, (1 R,2R)-3-(3-dimethylamino-1-ethyl-2-methylpropyl)-phenol, (2R,3R)-1-dimethylamino-3(3-methoxyphenyl)-2-methylpentan-3-ol, 6-dimethylaminomethyl-1-(3-methoxyphenyl)-cyyclohexan-1,3-diol, (1R,2R)-3-(2-dimethylaminomethylcyclohexyl)-phenol, und their physiologically acceptable salts or prodrugs.

9. The system according to claim 8, **characterized in that** said opioid is buprenorphine or one of its pharmaceutically acceptable salts or prodrugs.

10. The system according to any one of the previous claims, **characterized in that** the average force which is necessary to peel a 4 cm wide system orthogonally from a flat stainless steel surface at a speed of 50 mm/min is at least 1.0 N/4cm is, preferably at least 2.0 N/4cm und particularly at least 3.5 N/4cm.

11. The system according to any one of the previous claims, **characterized in that** said carrier layer (T) has a layer thickness ranging from 5.0 µm to 125 µm or from 125 µm 2000 µm.

12. The system according to any one of the previous claims, **characterized in that** said carrier layer (T) forms one of the two surface layers of said system.

13. The system according to any one of the previous claims, **characterized in that** said pressure-sensitive adhesive, on which said adhesive layer (K) is based, contains a polymer selected from the group consisting of polyacrylates, polyisobutylene, and silicones.

14. The system according to any one of the previous claims, **characterized in that** said adhesive layer (K) contains at least one adjuvant for the improvement of the transdermal permeation of the analgesic, the adjuvant being selected from the group consisting of surfactants, amines, inorganic bases, polyvinyl pyrrolidone, propylene glycol, benzyl alcohol, menthol, isosorbid nitrate, dodecylazacycloheptan-2-one, lactic acid, and ethanol.

15. A process for the production of a transdermal therapeutic system according to any one of the previous claims, comprising the following steps:
(a) mixing the constituents of the adhesive layer (K), optionally in a suitable solvent;
(b) applying the mixture obtained in step (a) onto one of the two surfaces of the carrier layer (T);
(c) optionally drying the mixture applied in step (b); and
(d) optionally applying the protective coating (S) to the mixture applied in step (b) and optionally dried in step (c).

16. The process according to claim 15, **characterized in that** in step (b) said mixture is applied with the assistance of a film drawing apparatus, the gap width of the doctor blade being in the range of from 10 to 50 µm.

17. The process according to claim 16, **characterized in that**, in step (b), the rate of application ranges from 1.0 to 25 mm/s.

18. The process according to claim 17, **characterized in that** the rate of application is selected such that the ratio of the gap width of said doctor blade to the layer thickness of the mixture applied in step (b), optionally after drying said mixture in step (c), is at least 1:1.

19. The process according to any one of claims 15 to 18, **characterized in that** the mixture produced in step (a) has a viscosity ranging from 1,000 to 10,000 mPa•s.

20. The process according to any one of claims 15 to 19, **characterized in that** the mixture produced in step (a) contains an organic solvent having a boiling point of less than 100 °C under ambient pressure.

## Revendications

1. Système thérapeutique transdermique pour l'administration d'un analgésique, comprenant
- une couche support (T) ;
- une couche adhésive (K), qui est immédiatement adjacente à la couche support (T), qui est à base d'un adhésif sensible à la pression et qui contient l'analgésique ; et
- le cas échéant une couche de protection amovible (S) ;
l'épaisseur de la couche adhésive (K) étant inférieure à 7,0 µm et la couche adhésive (K) contenant l'analgésique en une quantité supérieure à 0,60 g/m², par rapport à la surface de la couche adhésive (K).

2. Système selon la revendication 1, **caractérisé en ce que** la couche adhésive (K) contient l'analgésique en une quantité supérieure à 0,80 g/m², par rapport à la surface de la couche adhésive (K).

3. Système selon la revendication 1 ou 2,
**caractérisé en ce qu'**après l'application de la couche adhésive (K) en un endroit approprié de la peau d'un patient, en 72 heures, au moins 20 % en poids, de préférence au moins 50 % en poids, de la quantité d'analgésique contenue au départ dans la couche adhésive (K) sont libérés.

4. Système selon l'une quelconque des revendications précédentes, **caractérisé en ce que** la couche adhésive (K) contient l'analgésique en une quantité de 0,1 à 25 % en poids par rapport au poids total de la couche adhésive (K).

5. Système selon l'une quelconque des revendications précédentes, **caractérisé en ce que** la concentration de l'analgésique dans la couche adhésive (K) se situe dans la plage de 90 % à 100 % de sa concentration de saturation.

6. Système selon l'une quelconque des revendications précédentes, **caractérisé en ce que** l'épaisseur de la couche adhésive (K) est supérieure à 2,0 µm et inférieure à 5,0 µm.

7. Système selon l'une quelconque des revendications précédentes, **caractérisé en ce que** l'analgésique est un opiacé.

8. Système selon la revendication 7, **caractérisé en ce que** l'opiacé est choisi dans le groupe formé par le fentanyl, l'alfentanil, le sufentanil, la phénopéridine, l'aniléridine, le rémifentanil, la morphine, la diacétylmorphine, l'hydromorphone, la nicomorphine, l'oxycodone, la diamorphine, l'éthylmorphine, la cétobémidone, la péthidine, le dextromoramide, le piritramide, le dextropropoxyphène, le bézitramide, la méthadone, la pentazocine, la phénazocine, la buprénorphine, le butorphanol, la nalbuphine, la tilidine, la dézocine, le meptazinol, le (1R,2R)-3-(3-diméthylamino-1-éthyl-2-méthylpropyl)-phénol, le (2R,3R)-1-diméthylamino-3-(3-méthoxyphényl)-2-méthylpentan-3-ol, le 6-diméthylaminométhyl-1-(3-méthoxyphényl)-cyclohexane-1,3-diol, le (1R,2R)-3-(2-diméthylaminométhylcyclohexyl)-phénol et leurs sels ou précurseurs de médicament physiologiquement acceptables.

9. Système selon la revendication 8, **caractérisé en ce que** l'opiacé est la buprénorphine, un de ses sels ou précurseurs de médicament pharmaceutiquement acceptables.

10. Système selon l'une quelconque des revendications précédentes, **caractérisé en ce que** la force moyenne qui est nécessaire pour retirer un système d'une largeur de 4 cm, à une vitesse de 50 mm/min orthogonalement d'une surface plane en acier noble est d'au moins 1,0 N/4cm, de préférence d'au moins 2,0 N/4cm et en particulier d'au moins 3,5 N/4cm.

11. Système selon l'une quelconque des revendications précédentes, **caractérisé en ce que** la couche support (T) présente une épaisseur de couche dans la plage de 5,0 à 125 µm ou de 125 à 2000 µm.

12. Système selon l'une quelconque des revendications précédentes, **caractérisé en ce que** la couche support (T) forme une des deux couches superficielles du système.

13. Système selon l'une quelconque des revendications précédentes, **caractérisé en ce que** l'adhésif sensible à la pression, sur lequel est basée la couche adhésive (K), contient un polymère qui est choisi dans le groupe constitué par les polyacrylates, le polyisobutylène et les silicones.

14. Système selon l'une quelconque des revendications précédentes, **caractérisé en ce que** la couche adhésive (K) contient au moins une substance auxiliaire pour améliorer la perméation transdermique de l'analgésique, la substance auxiliaire étant choisie dans le groupe formé par les agents tensioactifs, les amines, les bases inorganiques, la polyvinylpyrrolidone, le propylèneglycol, l'alcool benzylique, le menthol, le nitrate d'isosorbide, la dodécylazacycloheptan-2-one, l'acide lactique et l'éthanol.

15. Procédé pour la préparation d'un système thérapeutique transdermique selon l'une quelconque des revendications précédentes, comprenant les étapes
(a) mélange des constituants de la couche adhésive (K), le cas échéant dans un solvant approprié ;
(b) application du mélange contenu dans l'étape (a) sur une des deux surfaces de la couche support (T) ;
(c) le cas échéant, séchage du mélange appliqué dans l'étape (b) ; et
(d) le cas échéant, application de la couche de protection (S) sur le mélange appliqué dans l'étape (b) et le cas échéant séché dans l'étape (c).

16. Procédé selon la revendication 15, **caractérisé en ce que** dans l'étape (b) le mélange est appliqué à l'aide d'un appareil de tirage d'un film, la largeur de la fente de la racle étant située dans la plage de 10 à 50 µm.

17. Procédé selon la revendication 16, **caractérisé en ce que** dans l'étape (b) la vitesse d'application se situe dans la plage de 1,0 à 25 mm/s.

18. Procédé selon la revendication 17, **caractérisé en ce que** la vitesse d'application est choisie de manière telle que le rapport de la largeur de fente de la racle à l'épaisseur de couche du mélange appliqué dans l'étape (b), le cas échéant après séchage du mélange dans l'étape (c) est d'au moins 1:1.

19. Procédé selon l'une quelconque des revendications 15 à 18, **caractérisé en ce que** le mélange préparé dans l'étape (a) présente une viscosité dans la plage de 1000 à 10 000 mPa.s.

20. Procédé selon l'une quelconque des revendications 15 à 19, **caractérisé en ce que** le mélange préparé dans l'étape (a) contient un solvant organique qui présente, à la pression atmosphérique, un point d'ébullition inférieur à 100°C.
